# EUROPÄISCHE PATENTSCHRIFT

(11) **EP 1 327 138 B1**
(45) Veröffentlichungstag und Bekanntmachung des Hinweises auf die Patenterteilung: **29.08.2012**
(21) Anmeldenummer: 01986566.6
(22) Anmeldetag: 02.10.2001
(51) Int. Cl.: G01N 27/417, F01N 11/00, G01N 33/00

(54) **VERFAHREN UND VORRICHTUNG ZUR EIGENDIAGNOSE EINES NOX-SENSORS**
METHOD AND DEVICE FOR THE ON-BOARD DIAGNOSIS OF AN NOX SENSOR
PROCEDE ET DISPOSITIF D'AUTODIAGNOSTIC D'UN CAPTEUR DE NOX

(30) Priorität: 07.10.2000 DE 10049685
(43) Veröffentlichungstag der Anmeldung: 16.07.2003
(73) Patentinhaber: Volkswagen Aktiengesellschaft, 38440 Wolfsburg (DE)
(72) Erfinder: SCHULZE, Frank, 38533 Vordorf (DE); DRÜCKHAMMER, Jens, 38108 Braunschweig (DE); LANG, Axel, 38302 Wolfenbüttel (DE); KRÖGER, Michael, 38465 Brome (DE)
(74) Vertreter: von Biedersee, Heidereich
(86) Internationale Anmeldenummer: PCT/EP2001/011386
(87) Internationale Veröffentlichungsnummer: WO 2002/031486

(56) Entgegenhaltungen:
- EP-A- 0 887 640
- EP-A- 0 892 265
- DE-A- 4 434 197
- DE-A- 19 945 374
- US-A- 5 558 752
- US-A- 5 804 700

## Beschreibung

Die Erfindung betrifft ein Verfahren und eine Vorrichtung zur Eigendiagnose eines NOX - Sensors mit den in den Oberbegriffen der unabhängigen Ansprüche genannten Merkmalen.

### Stand der Technik

Zur Verringerung des Schadstoffausstoßes von Kraftfahrzeugen ist in den letzten Jahren die sogenannte On- Board- Diagnose (OBD) vorgeschlagen und in einer zunehmenden Anzahl von Ländern auch durch gesetzliche Vorschriften für neu zugelassene. Kraftfahrzeuge eingeführt worden. Die OBD II, die seit 1995 in den USA schrittweise durchgesetzt wurde, verlangt eine Überwachung sämtlicher emissionsrelevanten Bauteile, insbesondere von Katalysator, Lambda-Sonde, Abgasrückführung, Tankentlüftung, Verbrennungsaussetzer, Testerschnittstelle, Manipulationsschutz und Sekundärluftsystem. Bei Fehlfunktionen oder Ausfällen der vom OBD II überwachten Bauteile wird eine Diagnoselampe angesteuert und ein Fehlercode gespeichert, so dass vom Führer des Kraftfahrzeuges bzw. einer Werkstatt Maßnahmen zur Behebung des Ausfalls bzw. der Fehlerfunktionen eingeleitet werden können.

Zur Erfüllung der gesetzlichen Abgasvorschriften ist bei modernen Verbrennungsmotoren, die im Mager- und Schichtladebetrieb einen geringeren Kraftstoffverbrauch aufweisen, eine zusätzliche Nachbehandlung von NOX -Emissionen unumgänglich. Bevorzugt wird zur Lösung dieses Problems ein NOX - Speicherkatalysator in der Abgasanlage eingesetzt. Um eine hohe Emissionsstabilität des Motors zu erreichen, wird dem NOX -Speicherkatalysator ein NOX -Sensor nachgeschaltet, mit dem insbesondere eine präzise Regelung des Mager- und NOX - Regenerationsbetriebs möglich ist.

Im Sinne der oben dargestellten On- Board- Diagnose-Anforderungen wird auch eine Überwachung der Funktion des NOX -Sensors gefordert. Aus der EP 0892265 A1 ist in diesem Zusammenhang bereits ein Gas-Sensor für die Messung von Gas-Oxyden bekannt, bei dem Abgas zur Messung in ein Doppel-Diffusionskammersystem geführt wird. Die Diffusionskammem weisen für die Messung Nernst-Zellen auf. Während in der ersten Diffusionskammer Sauerstoffmoleküle dem Gasgemisch entzogen werden, wird in der zweiten Diffusionskammer das zu messende Gas-Oxyd, beispielsweise Stickoxyd, in Stickstoff und Sauerstoff zerlegt. Eine an die erste Kammer angelegte Pumpzellenspannung wird auf einen konstanten Wert geregelt, der einer konstanten Sauerstoffkonzentration in dieser Kammer entspricht. Zur Eigendiagnose dieses Sensors wird überprüft, ob die auf einen konstanten Wert zu regelnde Sauerstoffkonzentration in der ersten Diffusionskammer nach Beendigung eines Aufwärmprozesses innerhalb eines vorgeschriebenen Bereiches liegt. Ist dies nicht der Fall, wird auf einen Fehler geschlossen. Als mögliche Fehler in der Funktionsweise des NOX -Sensors werden in der EP 0892265A1 Fehlfunktionen einer Haupt- oder Hilfspumpzelle, Leitungsunterbrechungen zu einem NOX -Sensorcontroller oder einer Heizungseinrichtung sowie Fehlfunktionen von Sensorelektroden angeführt. Aus der EP 0887640 A1 ist ferner eine weitere Eigendiagnosefunktion für einen derartigen Gas-Sensor beschrieben, bei der ein für die Sensor-Temperatur charakteristischer Innenwiderstand des Gas-Sensors überwacht wird. Falls der betreffende Innenwiderstand nicht innerhalb einer vorgegebenen Zeit einen vorgegebenen Wert erreicht, wird gemäß dem bekannten Verfahren auf einen Fehler des Gas-Sensors geschlossen.

Nachteilig bei den bekannten Verfahren ist, dass eine differenzierte Diagnose des Sensors mit der verschiedene mögliche Fehlerursachen unterschieden werden können, nur beschränkt möglich ist, da jeweils nur eine der ersten Diffusionskammer zugeordnete Pumpzellenspannung bzw. eine dem Innenwiderstand äquivalente Spannung auf die Einhaltung von Spannungsbereichen überwacht werden. Dies erschwert Wartung, Reparatur und präventive Maßnahmen zur Vermeidung von Fehlern.

### Zusammenfassung der Erfindung

Die Aufgabe der vorliegenden Erfindung besteht in der Angabe eines Verfahrens und einer Vorrichtung zur Eigendiagnose eines vorzugsweise in der Abgasanlage eines Verbrennungsmotors angeordneten NOX -Sensors, mit dem eine differenzierte und sichere Fehlerdiagnose erreicht werden kann.

Diese Aufgabe wird jeweils mit den Merkmalen der unabhängigen Ansprüche gelöst.

Gemäss der Erfindung werden bei einem in der Abgasanlage eines Verbrennungsmotors angeordneten NOX -Sensor Werte eines den Lambda-Wert des Abgases charakterisierenden Lambdasignals ermittelt. Der NOX -Sensor weist zur Messung der NOX -Konzentration eines Abgases eine Regeleinrichtung zur Regelung eines Sauerstoffpartialdrucks und eine ein die NOX -Konzentration im Abgas charakterisierendes NOX -Signal zur Verfügung stellende NOX -Messeinrichtung auf. Ermittelte Werte des Lambdasignals werden mit bei vorgegebenen Betriebsparametern plausiblen Lambda-Werten des Abgases verglichen und in Abhängigkeit von dem Vergleichsergebnis ein NOX -Sensor-Diagnosesignal gebildet. Damit wird eine differenzierte und sichere Fehlerdiagnose des NOX -Sensors möglich, da die von dem NOX -Sensor gelieferten Informationen über den Lambda-Wert des Abgases die momentane Arbeitsweise des NOX -Sensors wiederspiegeln während die zum Vergleich herangezogenen plausiblen Werte des Lambda-Wertes auf von der Arbeitsweise des NOX -Sensors unabhängigen Messungen anderer Sensoren oder Modellierungen des Lambda-Werts basieren.

weiterhim werden die Werte des NOX - Signals mit bei vorgegebenen Betriebsparametern plausiblen Werten der NOX - Konzentration verglichen und in Abhängigkeit von dem Vergleichsergebnis ein zwetes NOX - Sensor-Diagnosesignal gebildet.

Der NOX -Sensor weist in einer bevorzugten Form der Erfindung zwei zur Messung der NOX -Konzentrationen im Abgas zusammenwirkende Diffusionskammern auf, wobei die erste Diffusionskammer eine Sauerstoff-Messpumpzelleneinrichtung zur Regelung des Sauerstoffpartialdrucks in der ersten Diffusionskammer und die zweite Diffusionskammer eine NOX -Messpumpzelleneinrichtung aufweist, welche ein die NOX -Konzentration im Abgas charakterisierendes NOX -Signal zur Verfügung stellt. Beide Messpumpzellen arbeiten vorzugsweise nach dem Nemst-Prinzip. Erfindungsgemäß wird mittels der Sauerstoff-Messpumpzelleneinrichtung ein den Lambda-Wert des Abgases charakterisierendes Lambdasignal ermittelt. Die ermittelten Werte des Lambdasignals werden mit bei vorgegebenen Betriebsparametern plausiblen Lambda-Werten des Abgases verglichen und in Abhängigkeit von dem Vergleichsergebnis ein NOX -Sensor-Diagnosesignal gebildet.

Die Genauigkeit und Differenziertheit der Diagnose wird weiter erhöht, wenn ein auf einen konstanten Wert geregeltes Regelsignal, gegebenenfalls vorzugsweise der ersten Diffusionskammer, ermittelt, der ermittelte Wert mit bei vorgegebenen Betriebsparametern plausiblen Werte verglichen und ein weiteres entsprechendes NOX -Sensor-Diagnosesignal gebildet wird.

Eine Erhöhung von Genauigkeit und Differenziertheit der Diagnose wird auch dann erreicht, wenn bei einem NOX -Sensor mit einer Heizungseinrichtung und einer dieser zugeordneten Temperaturmesseinrichtung, die Werte eines Temperatursignals der Temperatur-Messeinrichtung mit bei vorgegebenen Betriebsparametern plausiblen Werten der NOX -Sensor-Temperatur verglichen und in Abhängigkeit von dem Vergleichsergebnis ein Temperatursignal-Diagnosesignal gebildet werden.

Einen Fehler im Bereich der Heizungseinrichtung und/oder der Temperatur-Messeinrichtung kann man insbesondere dann auf eine einfache Weise detektieren, wenn die Heizungseinrichtung eine Regelung aufweist und wenn überwacht wird, ob nach einer vorgegebenen Regelungszeit die Werte des Temperatursignals einer unterhalb einer vorgegebenen Betriebstemperatur des NOX -Sensors liegenden Temperatur entsprechen.

Bei einer besonders einfachen Ausführungsform der Erfindung arbeitet die Temperatur-Messeinrichtung auf der Basis der Ermittlung eines Innenwiderstandes des NOX - Sensors.

Eine besonders umfassende Diagnose wird erreicht, wenn der Vergleich der Werte des Lambda-, NOX -, Pumpzellen- oder Temperatursignals bei nicht beheiztem und bei beheiztem NOX -Sensor erfolgt.

Wenn bei dem Vergleich der Werte des Lambda-, NOX -, Regel- oder Temperatursignals eine Überwachung daraufhin erfolgt, ob vorgegebene Maximum-, Minimum- oder Toleranzbandwerte eingehalten werden, kann besonders einfach ein Kurzschluss zum Potential der Spannungsversorgung bzw. zur Masse bzw. eine Leitungsunterbrechung detektiert werden.

Bei einer weiteren Ausführungsform der Erfindung wird für vorgegebene Werte von Betriebsparametern für ein vorgegebenes Zeitintervall die Bildung eines Diagnosesignals ausgesetzt. Damit ist es möglich, falsche Fehlermeldungen zu vermeiden. Ferner ist zur Vermeidung von falschen Fehlermeldungen bei einer weiteren Ausführungsform der Erfindung vorgesehen, bestimmten Bedingungen vorausgesetzt, gesetzte Fehlersignale zu heilen. Vorzugsweise wird diese Heilung an das Vorliegen von vorgegebenen Betriebsparametern des Verbrennungsmotors geknüpft.

Die Effizienz von Wartungs- und Reparaturmaßnahmen wird erhöht, wenn gemäß einer weiteren Ausführungsform der Erfindung NOX -Sensor-Diagnosesignale, die ermittelten Fehlern entsprechen, vorzugsweise zusammen mit den Werten der betreffenden Betriebsparameter gespeichert werden.

Eine erhöhte Sicherheit gegenüber Fehlalarmen wird erreicht, wenn nur bei nicht geheilten Fehlersignalen eine Signallampe angesteuert wird.

Die erfindungsgemäße Vorrichtung zur Eigendiagnose eines in der Abgasanlage eines Verbrennungsmotors angeordneten NOX -Sensors dient der Ausführung des erfindungsgemäßen Verfahrens und ermöglicht eine genauere und sichere Kontrolle der NOX -Emissionen eines Verbrennungsmotors.

Bei einer bevorzugten Ausführungsform der Vorrichtung weist die Abgasanlage einen NOX -Speicherkatalysator und optional stromaufwärts dieses NOX - Speicherkatalysators einen Vorkatalysator auf. NOX -Sensoren können stromabwärts und/oder stromaufwärts des NOX -Speicherkatalysators angeordnet sein. Eine derartige Vorrichtung verbessert den umweltfreundlichen und sicheren Betrieb eines Verbrennungsmotors.

Weitere Merkmale und Vorteile der vorliegenden Erfindung ergeben sich aus den abhängigen Ansprüchen sowie unabhängig von ihrer Zusammenfassung in den Ansprüchen aus der nachfolgenden Beschreibung bevorzugter erfindungsgemäßer Ausführungsbeispiele in Verbindung mit den zugehörigen Zeichnungen.

Die Zeichnungen zeigen in schematischer Darstellung:
Figur 1 einen Verbrennungsmotor mit Abgasanlage
Figur 2 Komponenten eines Motorsteuergerätes
Figur 3 einen Doppelkammer -NOX -Sensor
Figur 4 typische Messsignalverläufe eines NOX -Sensors
Figur 5 Überwachungsbereiche eines NOX -Signals
Figur 6 Überwachungsbereiche eines Breitband-Lambdasignals
Figur 7 Überwachungsbereiche eines Lambda-Sprungsignals
Figur 8 Überwachungsbereiche eines Pumpzellensignals
Figur 9 Überwachungsbereiche eines Temperatursignals.

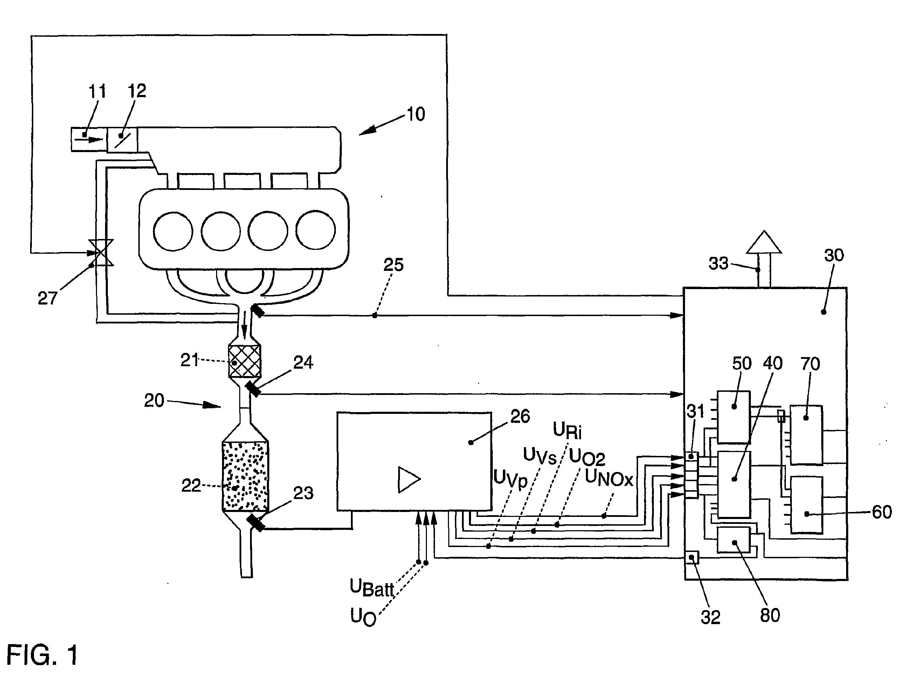

Dem in Figur 1 schematisch dargestellten Verbrennungsmotor 10 eines Kraftfahrzeuges ist eine Abgasanlage 20 nachgeschaltet, die einen Vorkatalysator 21, vorzugsweise ein konventioneller 3-Wege-Katalysator, und einen NOX -Speicherkatalysator 22 aufweist. Zur Steuerung bzw. Regelung des Verbrennungsmotors 10 ist ein Motorsteuergerät 30 vorgesehen, welches u.a. eine Diagnoseeinrichtung 40 für die Eigendiagnose eines stromabwärts des NOX -Speicherkatalysator 22 angeordneten NOX -Sensors 23 beinhaltet. Weitere NOX -Sensoren können stromaufwärts des NOX - Speicherkatalysators 22 angeordnet sein. Die Abgasanlage 20 weist ferner eine vorzugsweise lineare Lambda-Sonde 25 zur Erfassung des Lambda-Wertes des Abgases stromaufwärts des Vorkatalysators 21 sowie einen Temperatursensor 24 zur Messung der Abgastemperatur stromabwärts des Vorkatalysators 21 auf. An die Abgasanlage 20 ist ein Abgasrückführungssystem 27 angeschlossen.

Bei dem NOX -Sensor 23 handelt sich um einen im folgenden noch genauer dargestellten beheizbaren Doppelkammer -NOX -Sensor, dessen Ausgangssignale einem NOX -Sensor-Controller 26 zugeführt werden, welcher UO2 UVP, UNOX, UVS, URI Signale abgibt, die über zugeordnete Signaleingänge 31 dem Motorsteuergerät 30 zugeführt werden. Am NOX -Sensor-Controller 26 liegt eine Versorgungsspannung UBatt an. Das Massepotential ist mit UO bezeichnet. Vorzugsweise ist der NOX - Sensor-Controller 26 in räumlicher Nähe zum NOX -Sensor 23 angeordnet, damit nur kurze Signalleitungswege zwischen beiden erforderlich sind. Die vom NOX -Sensor-Controller 26 ausgegebenen Signale werden über Leitungen bzw. einen Kabelbaum in die Messsignaleingänge 31 des Motorsteuergerätes 30 gespeist. Von den Messsignaleingängen 31 werden die erwähnten Signale der Diagnoseeinrichtung 40 zugeführt. Das Motorsteuergerät 30 weist als weitere Komponenten eine NOX -Sensor-Signalaufbereitungseinrichtung 50, eine NOX -Speicherkatalysator-Diagnoseeinrichtung 60 und eine NOX -Speicherkatalysator-Regelungseinrichtung 70 auf. Vorzugsweise wird ein temperaturgeregelter NOX -Sensor 23 verwendet, dessen Regelung durch eine NOX -Sensor-Heizungseinrichtung 80 erfolgt, welche über einen Anschluss 32 mit dem NOX -Sensor-Controller 26 verbunden ist.

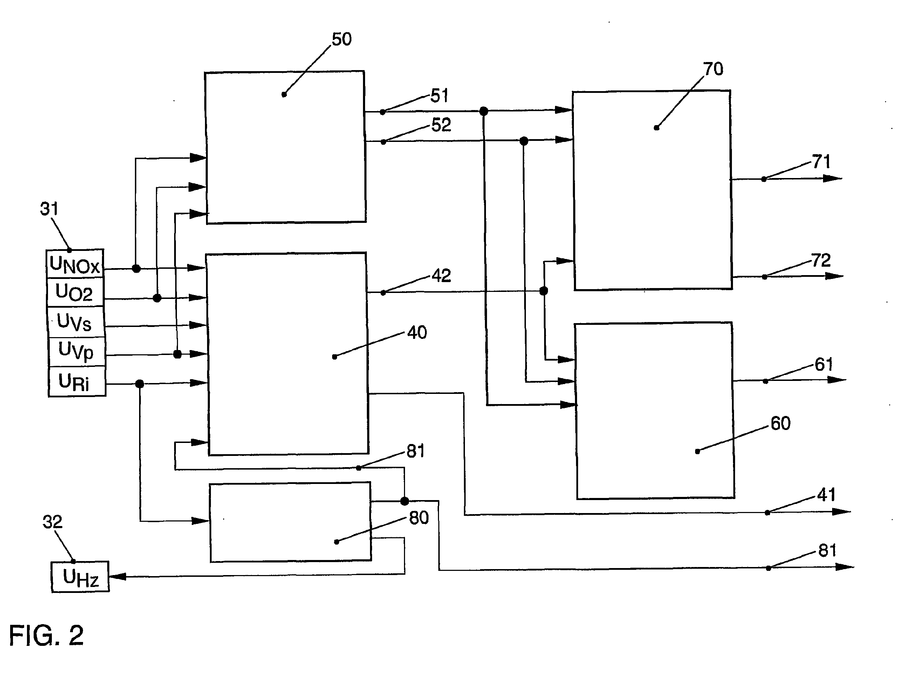

Figur 2 zeigt eine schematische Darstellung der Signalwege zwischen den Komponenten des Motorsteuergerätes 30. Von der NOX -Sensor Signalaufbereitungseinrichtung 50 wird ein NOX -Massenstromsignal 52 errechnet und der NOX -Speicherkatalysator-Diagnoseeinrichtung 60 und der NOX - Speicherkatalysator-Regeleinrichtung 70 zugeführt. Femer wird ein NOX - Konzentrationssignal 51 den beiden Einrichtungen 60 und 70 von der NOX -SensorSignal-Aufbereitungseinrichtung 50 zugeführt. Wird von der NOX -Speicherkatalysator-Diagnoseeinrichtung 60 ein Fehler detektiert, wird ein Fehlersignal 61 ausgegeben. Die NOX -Speicherkatalysator-Regeleinrichtung 70 erzeugt ein Soll-Lambdasignal 71 sowie ein NOX -Speichersignal 72, mit dem eine Regeneration des NOX -Speicherkatalysators 22 angefordert werden kann.

Betriebsparameter des Verbrennungsmotors 10 wie Abgastemperatur, Last, Drehzahl, Rohemissionsverlauf oder dergleichen werden von dem Motorsteuergerät 30 in an sich bekannter Weise als Signal des NOX -Sensors 23, des Temperatur-Sensors 24, der Lambda-Sonde 25 sowie weiterer (nicht dargestellter) Sensoren erfaßt. Über Stellglieder, wie beispielsweise eine Drosselklappe 12 in der Luftzuführung 11 des Verbrennungsmotors 10 oder das Abgasrückführsystem 27, werden Betriebsparameter des Verbrennungsmotors 10 vom Motorsteuergerät 30 beeinflusst. Die Kommunikation zwischen dem Motorsteuergerät 30 und dem Verbrennungsmotor 10 bzw. den Stellgliedern erfolgt über ein Kabel- oder Bussystem 33.

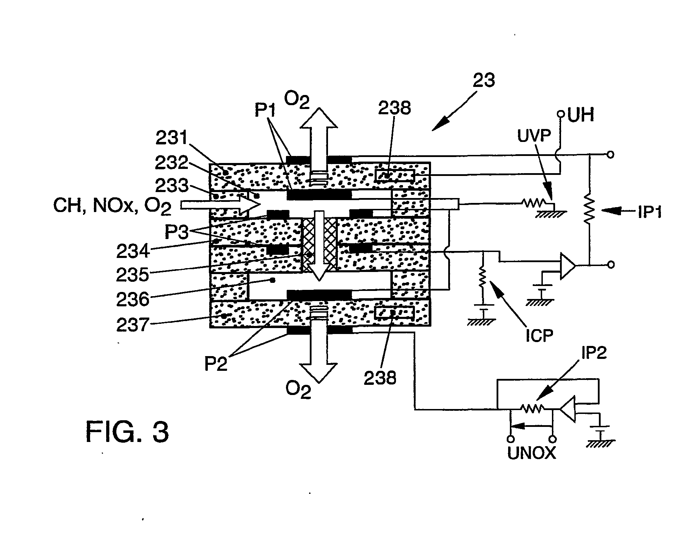

Der in Figur 3 dargestellte NOX -Sensor zur Messung der NOX -Konzentration im Abgas ist als Doppelkammersensor mit einer ersten und zweiten Diffusionskammer 232, 236 ausgebildet. Über eine Diffusionsbarriere 233 kann ein Teil des Abgases mit NOX -, 02-und weiteren Komponenten die erste Diffusionskammer 232 erreichen. Die Diffusionskammer 232 weist eine nach dem Nernst-Prinzip arbeitende Sauerstoff-Messpumpzelle 231 mit Pumpelektroden P1 auf. Mittels letzterer kann der Sauerstoffgehalt in der Diffusionskammer 232 verändert werden. Über eine weitere Diffusionsbarriere 235 gelangt Abgas in die zweite Diffusionskammer 236. Diese weist eine ebenfalls nach dem Nernst-Prinzip arbeitende NOX -Messpumpzelle 237 mit Pumpelektroden P2 auf. Die im Abgas enthaltenen Stickoxyde werden durch ein spezielles Material der inneren P2 Elektroden katalytisch in die Komponenten N2 und O2 zerlegt. Zur Kalibrierung des Systems wird, wie an sich bereits bekannt ist, eine 02-Referenzzelle 234 mit Elektroden P3 verwendet.

Die Arbeitsweise des NOX -Sensors 23 ermöglicht es, dem Lambdawert des Abgases zugeordnete Lambdasignale zu ermitteln. An die Sauerstoff-Messpumpzelle 231 wird eine Pumpspannung UVS angelegt. Der Strom ICP wird so geregelt, dass ein konstanter, vorzugsweise stöchiometrischer Wert der Sauerstoff-Konzentration in der ersten Diffusionskammer 232 resultiert..Aus dem Pumpstrom IP1 wird ein Breitband-Lambdawert UO2 berechnet. Die Spannung UVP entspricht einem Lambdasprungsignal. Über den Pumpzellenstrom IP2 der Diffusionskammer 237 kann ein der NOX - Konzentration im Abgas entsprechendes Spannungssignal UNOX ermittelt werden.

Um die für eine NOX -Messung erforderliche Mindesttemperatur zu gewährleisten, weist der NOX -Sensor 23 Heizelemente 238 auf, denen eine Heizspannung UH von der Heizungseinrichtung 80 zugeführt werden kann. ist. Eine zugeordnete Temperatur-Messeinrichtung gibt ein Temperatursignal ab, aus dem die NOX -Sensor-Temperatur ermittelbar ist. Vorzugsweise erfolgt die Temperaturmessung in an sich bekannter Weise durch die Ermittlung eines NOX -Sensor-Innenwiderstandes RI. Als Temperatur-Messeinrichtung fungieren in diesem Fall der NOX -Sensor 23 selbst und der NOX - Controller 26, der aus einer Innenwiderstandsmessung ein den Innenwiderstand RI repräsentierendes Spannungssignal URI berechnet.

Das erfindungsgemäße Verfahren geht von der Idee aus, von dem NOX -Sensor 23 gelieferte, dem Lambda-Wert und der NOX -Konzentration des Abgases zugeordnete Informationen für die Eigendiagnose zu verwenden. Dazu werden die betreffen den vom NOX -Sensor gelieferten Signale mit plausiblen Werten verglichen. Die Diagnoseeinrichtung 40 weist dazu Vergleichsmittel zum Vergleich der Werte der Signale mit vorgegebenen Prüfmustem und zur Bildung eines Vergleichsergebnisses sowie Auswertungsmittel zur Bildung und Ausgabe eines entsprechenden NOX -Sensor-Diagnosesignals auf. Die Diagnoseeinrichtung 40 besteht vorzugsweise aus einem Mikrocontroller mit einer CPU, einem Programmspeicher, einem Datenspeicher sowie Eingabe- und Ausgabeschnittstellen. Bei Auftreten eines Fehlers wird ein Fehlersignal 41 abgegeben. Wird dagegen kein Fehler detektiert, wird von der Diagnoseeinrichtung 40 vorzugsweise ein Gültigkeitssignal 42 abgegeben, welches der NOX -Speicherkatalysator-Diagnoseeinrichtung 60 und der NOX -Speicherkatalysator-Regeleinrichtung 70 zugeführt wird.

Im folgenden wird das Verfahren zunächst für den Fall genauer erläutert, dass die Eigendiagnose auf der Auswertung von Lambdawertsignalen beruht. Die beispielsweise in einem ROM als Kennfeld abgelegten Prüfmuster repräsentieren plausible Werte von Lambdasignalen und werden abhängig von den Betriebsparametern des Verbrennungsmotors gewählt. Vorzugsweise errechnet der NOX -Controller 26 ein Breitband-Lambdasignal UO2 und/oder ein Lambda-Sprungsignal UVP, welche mit entsprechenden plausiblen Werten verglichen werden. Es versteht sich, dass zur Ermittlung der plausiblen Werte auch die von dem stromabwärts des Vorkatalysator angeordneten Lambda-Sensor 24 gelieferten Signale sowie Signale weiterer Sensoren herangezogen werden können.

Bei den Prüfmustem wird berücksichtigt, dass einerseits Fehler auftreten können, die durch Alterung oder Vergiftung des NOX -Sensors 23 entstehen. Beispiele hierfür sind Fehler in einer der Pumpzellen 231, 237 oder die Korrosion der Sensorelektroden P1, P2, P3. Andererseits können Fehler auftreten, die durch Kurzschlüsse, beispielsweise zur Batteriespannung UBatt, zur Masse UO oder durch Leitungsunterbrechung bei der Zuführung der Signale vom NOX -Sensor-Controller 26 zu den Signaleingängen 31 des Motorsteuergerätes 30 verursacht werden. In beiden Fällen führen die Fehler zu nicht plausiblen Werten der erwähnten Signale bei vorgegebenen Betriebsparametern des Verbrennungsmotors 10.

Ferner ist es zweckmäßig, verschiedene Betriebspunkte des Verbrennungsmotors 10, wie Schichtbetrieb, Homogenbetrieb, Homogenmagerbetrieb, Vollastbetrieb, Schubbetrieb und/oder NOX -Katalysator-Regeneration des Verbrennungsmotors zu berücksichtigen, da verschiedenen Betriebspunkten des Verbrennungsmotors 10 jeweils unterschiedliche Lambda-Werte des Abgases entsprechen. Im folgenden soll dies an einem Beispiel verdeutlicht werden.

Im Schubbetrieb, beispielsweise bei einem Fahrzeug auf einer Gefällstrecke, wird die Kraftstoffeinspritzung des Verbrennungsmotors 10 abgeschaltet. Die vom Verbrennungsmotor 10 angesaugte Luft wird dabei über Auslassventile in die Abgasanlage 20 geleitet, ohne dass dabei nennenswerte Änderungen der Sauerstoffkonzentration erfolgen. Das Lambdasignal des NOX -Sensors 23 wird nun mit dem Signal der Lambda-Sonde 25 verglichen. Diese würde eine maximale Sauerstoffkonzentration anzeigen. Würde der NOX -Sensor dagegen für ein vorgegebenes Zeitintervall ein Lambdasignal abgeben, welches Abgas mit niedrigerer Sauerstoffkonzentration entspricht, wäre dies ein nicht plausibler Wert relativ zu dem Signal der Lambda-Sonde 25. Es wird daher, wenn der Verbrennungsmotor im Schubbetrieb ist, ein diesem zugeordneter plausibler Lambda-Wert zum Vergleich herangezogen. Hierbei wird auch die Signaldynamik berücksichtigt. Beispielsweise wird, um zu berücksichtigen, dass die Abgasanlage eine endliche Gaslaufzeit besitzt, daher der dem Schubbetrieb zugeordnete Lambda-Wert mit einer Verzögerungszeit an einem stromabwärts des NOX -Katalysator angeordneten NOX -Sensor beobachtbar ist, wird für eine entsprechende Zeit die Ausgabe eines NOX -Sensor-Diagnosesignals ausgesetzt.

Zusätzlich zu einer auf der Überwachung des Lambdasignals basierenden Diagnose kann das Signal UNOX, sowie können ergänzend die Signale UVS und URI des NOX -Sensors überwacht werden. Dabei wird im Prinzip genauso vorgegangen, wie es für den Fall des Lambdasignals dargestellt wurde. Aus den Diagnosesignalen, die den einzelnen Signalen UO2 UVP, NOX, UVS und URI zugeordnet sind, kann dann durch Kombination ein Gesamt -NOX -Sensor-Diagnosesignal gebildet werden.

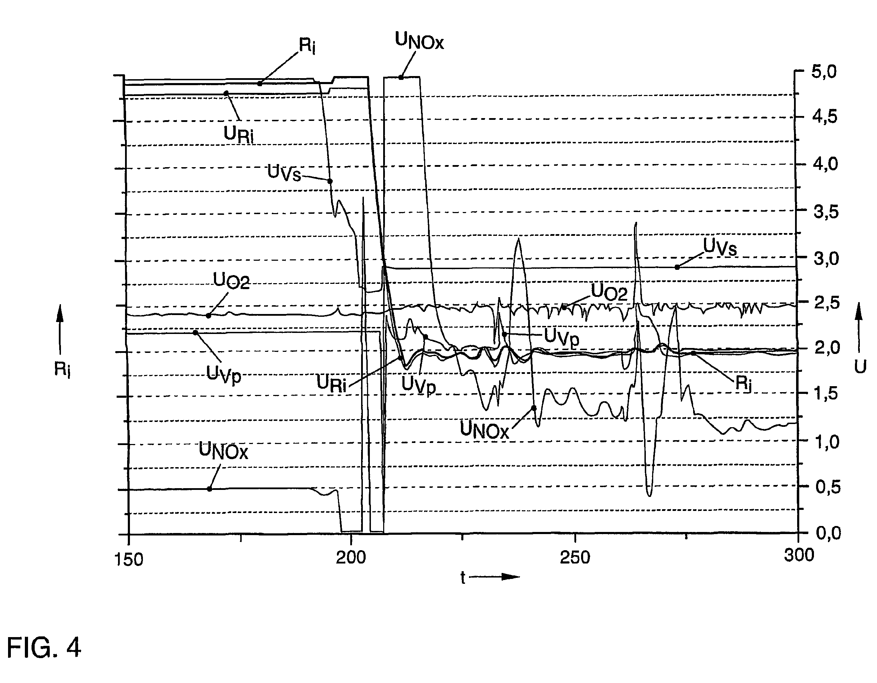

Ein besonders wichtiger Betriebsparameter ist die Temperatur des NOX -Sensors. Für die Messung der NOX -Konzentration des Abgases muss der Sensor eine Mindesttemperatur aufweisen. Figur 4 zeigt eine Prinzipdarstellung des zeitlichen Verlaufs der Signale UO2 UVP, UNOX, UVS und URI vor und nach einer Beheizung des Sensors. Vor dem Erreichen der Mindesttemperatur befinden sich die Werte der Signale i.w. auf einem konstanten Niveau. In Figur 4 bezeichnet RI den temperaturabhängigen Innenwiderstand eines NOX -Sensors mit negativer Temperaturcharakteristik. Ab einem Zeitwert von etwa 200 zeigt RI einen steilen Abfall. Von diesem Zeitpunkt an, der einem auf Betriebstemperatur beheizten NOX -Sensor 23 entspricht, weisen die erwähnten Signalwerte erhöhte Variationen gegenüber denen in dem vorherigen Zeitraum auf. Bei der Überwachung der erwähnten Signale des NOX - Sensors 23 wird vorzugsweise das unterschiedliche Verhalten der Signale vor und nach Beheizung des NOX -Sensors 23 berücksichtigt.

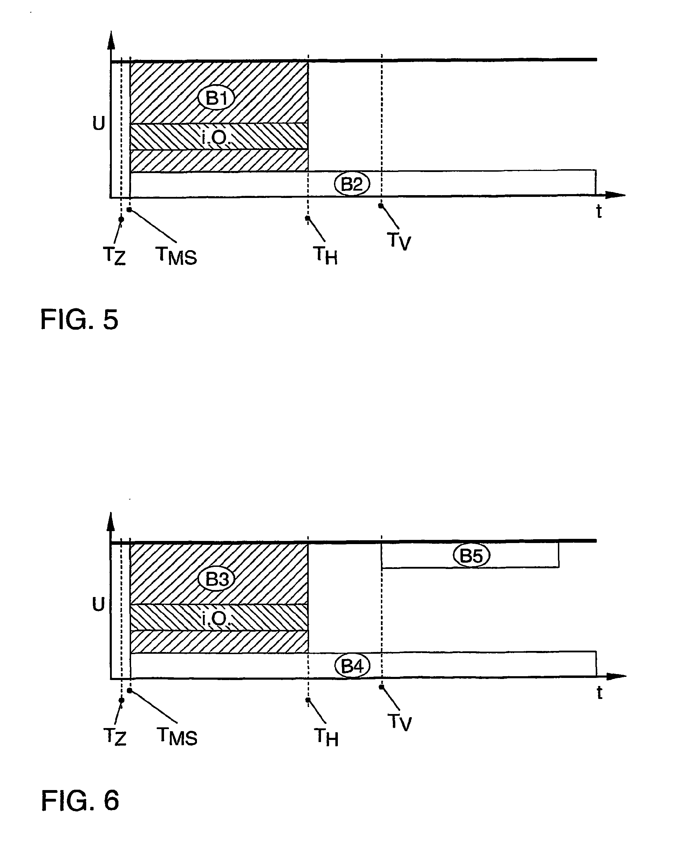

Im folgenden werden anhand der Figuren 5 bis 9 bevorzugte Bereiche der Spannungs-Überwachung der Signale UNOX, U02, UVP, UVS und URI dargestellt. Es versteht sich von selbst, dass die im folgenden beschriebenen Fälle nur Beispiele darstellen, die in keiner Weise eine Beschränkung der Erfindung implizieren sollen.

Figur 5 zeigt eine schematische Darstellung der Bereichsüberwachung des NOX - Signals UNOX. Zum Zeitpunkt TZ wird die Zündung des Verbrennungsmotors 10 eingeschaltet, danach zum Zeitpunkt TMS wird der Motor gestartet. Ab dem Zeitpunkt TH wird der NOX -Sensor 23 beheizt. Im Zeitintervall zwischen TMS und TH erfolgt eine Überwachung, ob das NOX -Signal innerhalb eines vorgegebenen Toleranzbandes B1 liegt. Die Abkürzung i.O. deutet an, dass in diesem Fall kein Fehler vorliegt. Von dem Zeitpunkt TMS an erfolgt eine Überwachung, ob das NOX -Signal kleiner als ein vorgegebener Minimalwert ist, was einem Kurzschluss nach Masse U0 entspricht. Diese Überwachung findet auch über den Zeitpunkt TH hinaus statt, d.h. wenn der NOX - Sensor 23 beheizt ist. In beiden Fällen wird vorzugsweise ein Fehler nur dann erkannt, wenn die Überschreitung der vorgegebenen Grenzen der Messsignale während eines vorgegebenen Zeitintervalls erfolgt. Wenn keine Überschreitung der vorgegebenen Grenzen bis zu einem Zeitpunkt TV vorliegt, wird ein die Gültigkeit des NOX -Signal anzeigendes Diagnosesignal gebildet. Femer kann auch überwacht werden, ob bei beheiztem NOX -Sensor 23 das Signal größer als ein vorgegebener Schwellwert ist. Darüber hinaus ist es vorteilhaft, die Überwachung nach Kurzschluss im Bereich B2 nur vorzunehmen, wenn eine Schubabschaltung des Verbrennungsmotors 10 mindestens um eine vorgegebene Zeit zurückliegt.

Figur 6 zeigt eine schematische Darstellung einer Bereichsüberwachung des Lambdasignals UO2 Die Bereiche B3 und B4 entsprechen den Bereichen B1 und B2 des NOX -Sensorsignals der Figur 5. Der Bereich B5 bezeichnet einen Bereich, in dem das Lambdasignal auf Überschreiten eines Maximalwertes hin überwacht wird.

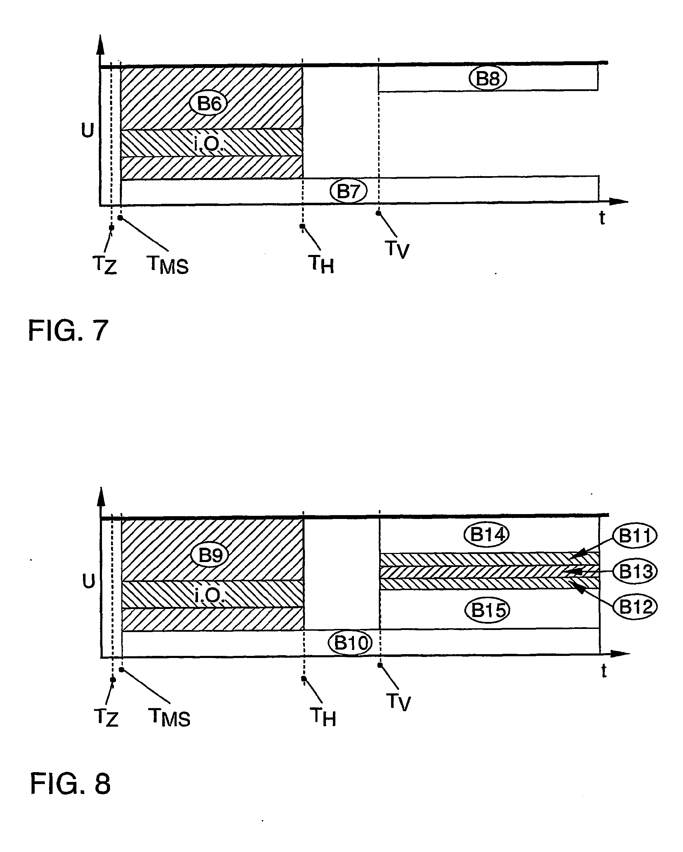

Figur 7 zeigt eine schematische Darstellung einer Bereichsüberwachung des Lambda-Sprungsignals UVP des NOX -Sensors 23 mit den Bereichen B6, B7 und B8, die den Bereichen B3, B4 und B5 der Figur 6 entsprechen.

Figur 8 zeigt eine schematische Bereichsüberwachung des Pumpzellensignals UVS. Die Bereiche B9 und B10 entsprechen einer Toleranzband- und einer Minimum-Fehlerüberwachung. Der Bereich B14 berücksichtigt einen Maximumfehler, beispielsweise durch einen Kurzschluss, nach UBatt. Die weiteren Bereiche B11, B12, B13 und B15 berücksichtigen, dass die Spannung UVS der ersten Messpumpzelle 231 sich nur in einem engen Toleranzband um einen gegebenen Spannungswert bewegen darf. Herstellungsbedingt treten zwischen verschiedenen NOX -Sensoren jedoch Wertstreuungen auf, die es erforderlich machen, diesen Spannungswert, zum Beispiel nach Austausch des NOX -Sensors, adaptiv neu anzulernen, wenn auf ein schmales Toleranzband hin diagnostiziert werden soll.

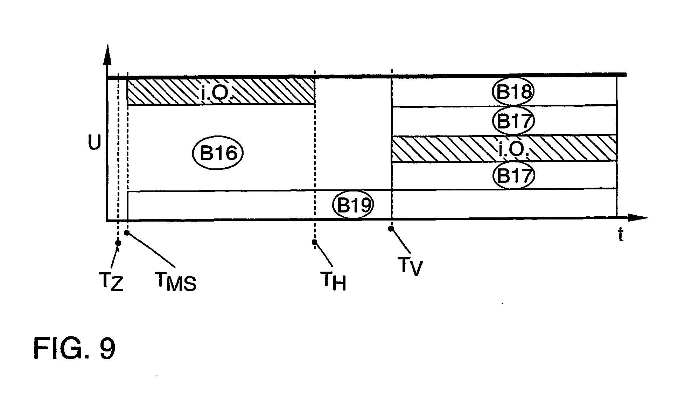

Figur 9 zeigt eine schematische Darstellung der Bereichsüberwachung des den NOX - Sensor-Innenwiderstand RI repräsentierenden Signals URI. Vorausgesetzt wird hier, das der Sensor-Innenwiderstand eine negative Temperatur-Charakteristik besitzt. Im Bereich B16, nach erfolgtem Motorstart und bevor der NOX -Sensor beheizt wird, wird geprüft, ob ein einer niedrigen Temperatur entsprechendes Temperatursignal vorliegt. Wie aus Figur 4 zu ersehen ist, ist der Istwert des Innenwiderstandes unmittelbar nach dem Start relativ hoch. In dem Fall jedoch, dass der Start nur kurz nach einem vorhergehenden Betrieb erfolgt ist, d.h. bei einem vorgewärmten Sensor, trifft dies nicht zu. Um dies zu berücksichtigen, kann zum Beispiel die Wassertemperatur eines Kühlsystems als Betriebsparameter zur Plausibilitätsprüfung herangezogen werden. Nachdem der Sensor vom Zeitpunkt TH an beheizt wird, wird in den Bereichen B17 geprüft, ob die Sensortemperatur in einem unzulässigen Werte-Band liegt. Dieser Bereich weist nach einer vorgegebenen Regelungszeit auf einen Fehler in der Temperaturregelung hin. Diesem Fall entspricht es, wenn die Heizungsregelung einen PI-Regler aufweist, der für eine vorgegebene Zeit am Anschlag ist. Ursache hierfür kann beispielsweise ein Kurzschluss der Heizspannung zur Masse oder nach UBatt, eine Leitungsunterbrechung oder ein defektes Heizelement sein. Um den Fall eines ausgekühlten Motors zu berücksichtigen, ist es in diesem Fall vorteilhaft, einen durch einen Außentemperatursensor gemessenen Außentemperaturwert zu heran zu ziehen und die Regelungszeit entsprechend zu verlängern. Femer kann im gesamten Bereich der Spannung U, einschließlich des Toleranzbandes i.O. überwacht werden, ob eine zeitliche konstante Abweichung von einem Soll-Wert länger als eine gegebene Regelungszeit auftritt. Dies entspricht ebenfalls einem Fehler in der Temperaturregelung. Der Bereich B18 beschreibt einen Fall, in dem der NOX -Sensor nicht beheizt wird, so daß der Widerstandswert einen Maximalwert überschreitet oder ein Kurzschluss nach der Versorgungsspannung vorliegt. Der Bereich B19 charakterisiert eine Unterschreitung eines vorgegebenen Minimalwerts des Sensor Widerstandswerts, eine Leitungsunterbrechung oder einen Kurzschluss nach Masse der des.

In Abhängigkeit von dem Wert des Diagnosesignals kann eine Heilung gesetzter Fehlersignale vorgenommen werden. Vorzugsweise wird hierbei auf das Auftreten bzw. Nicht-Auftreten von Fehlern während eines vorgegebenen Zeitintervalls abgestellt. Weiterhin können für die Heilung Bedingungen gesetzt werden, wie beispielsweise, dass der NOX -Sensor 23 beheizt ist, die Abgastemperatur sich innerhalb definierter Grenzen befindet oder die Heizungsregelung 80 kein Fehlersignal 81 abgibt. Femer kann die Bedingung gesetzt sein, dass der Wert des NOX -Sensor-Innenwiderstandes RI sich innerhalb eines Soll-Bandes befindet. Es kann dann vorgesehen sein, dass lediglich bei nicht geheilten Fehlersignalen eine Signallampe angesteuert wird.

Wenn ein Fehlersignal 41 gesetzt wird, kann dieses gespeichert werden, vorzugsweise zusammen mit den entsprechenden Betriebsparametern, um eine anschließende Fehlerdiagnose zu erleichtern. Diese Speicherung kann auch bei geheilten Fehlersignalen vorgenommen werden, um für Wartungs- und Reparaturarbeiten Informationen über sporadische Fehler zur Verfügung zu haben.

## Patentansprüche

1. Verfahren zur Eigendiagnose eines in einer Abgasanlage (20) eines Verbrennungsmotors (10) angeordneten NOX-Sensors (23), welcher zur Messung der NOX-Konzentration eines Abgases eine Regeleinrichtung zur Regelung eines Sauerstoffpartialdrucks in einer ersten Diffusionskammer (232) des NOx-Sensors (23) aufweist und die Regeleinrichtung ein den Lambda-Wert des Abgases charakterisierendes Lambdasignal (UO2, UVP) zur Verfügung stellt, und welcher eine NOX-Messeinrichtung aufweist, die ein die NOX-Konzentration im Abgas charakterisierendes NOX-Signal zur Verfügung stellt, wobei ermittelte Werte des Lambdasignals (UO2, UVP) mit bei vorgegebenen Betriebsparametern plausiblen Lambda-Werten des Abgases verglichen und in Abhängigkeit von dem Vergleichsergebnis ein erstes NOX-Sensor-Diagnosesignal gebildet wird, **dadurch gekennzeichnet, dass**
die Werte des NOX-Signals mit bei vorgegebenen Betriebsparametern plausiblen Werten der NOX-Konzentration verglichen werden und in Abhängigkeit von dem Vergleichsergebnis ein zweites NOX-Sensor-Diagnosesignal gebildet wird.

2. Verfahren nach Anspruch 1, **dadurch gekennzeichnet, dass** das Lambdasignal ein Breitband-Lambdasignal (UO2) oder ein Lambda-Sprungsignal (UVP) ist.

3. Verfahrens nach zumindest einem der vorhergehenden Ansprüche, **dadurch gekennzeichnet, dass** die Regeleinrichtung zur Regelung des Sauerstoffpartialdrucks als nach dem Nernst-Prinzip arbeitende Sauerstoff-Messpumpzellen-Einrichtung (231) ausgebildet ist.

4. Verfahren nach zumindest einem der vorhergehenden Ansprüche, **dadurch gekennzeichnet, dass** die NOX-Messeinrichtung als nach dem Nernst-Prinzip arbeitende NOX-Messpumpzellen-Einrichtung (237) ausgebildet ist.

5. Verfahren nach zumindest einem der Ansprüche 3 oder 4, **dadurch gekennzeichnet, dass** der NOX-Sensor (23) die erste Diffusionskammer (232) und eine zweite Diffusionskammer (236) aufweist, der jeweils eine der Messpumpzellen-Einrichtungen (231; 237) zugeordnet sind.

6. Verfahren nach zumindest einem der vorhergehenden Ansprüche, **dadurch gekennzeichnet, dass** ermittelte Werte eines auf einen konstanten Wert geregelten Regelsignals der ersten Diffusionskammer (232) mit bei vorgegebenen Betriebsparametern plausiblen Werten des Regelsignals verglichen werden und in Abhängigkeit von dem Vergleichsergebnis ein weiteres NOX-Sensor-Diagnosesignal gebildet wird.

7. Verfahren nach zumindest einem der vorhergehenden Ansprüche, **dadurch gekennzeichnet, dass** der NOX-Sensor (23) eine Heizungseinrichtung (238) mit einer zugeordneten Temperatur-Messeinrichtung aufweist, um den NOX-Sensor (23) bei einer vorgegebenen Temperatur zu betreiben, wobei die Temperatur-Messeinrichtung ein Temperatursignal abgibt, aus dem die NOX-Sensor-Temperatur ermittelbar ist, und wobei die Werte des Temperatursignals mit bei vorgegebenen Betriebsparametern plausiblen Werten der NOX-Sensor-Temperatur verglichen und in Abhängigkeit von dem Vergleichsergebnis ein Temperatur-Diagnosesiglal gebildet wird.

8. Verfahren nach Anspruch 7, **dadurch gekennzeichnet, dass** die Heizungseinrichtung (238) eine Temperaturregelung aufweist und dass, wenn nach einer vorgegebenen Regelungszeit der Wert des Temperatursignals einer unterhalb einer vorgegebenen Betriebstemperatur des NOX-Sensors (23) liegenden Temperatur entspricht, ein einen Fehler charakterisierendes Temperatur-Diagnosesignal gebildet wird.

9. Verfahren nach Anspruch 7 oder 8, **dadurch gekennzeichnet, dass** die Temperatur Messeinrichtung auf der Basis der Ermittlung eines Innenwiderstandes des NOX-Sensors (23) arbeitet.

10. Verfahren nach den Ansprüchen 1, 6 und 7, **dadurch gekennzeichnet, dass** die Werte des Lambdasignals, des NOX-Signals, des Regelsignals oder des Temperatursignals bei beheiztem und nicht beheiztem NOX-Sensor ermittelt werden.

11. Verfahren nach den Ansprüchen 1, 6 und 7, **dadurch gekennzeichnet, dass** zur Erkennung von Leitungsfehlern bei dem Vergleich der Werte des Lambdasignals, des NOX-Signals, des Regelsignals oder des Temperatursignals mit den jeweiligen plausiblen Werten eine Überprüfung daraufhin erfolgt, ob vorgegebene Maximum-, Minimum- oder Toleranzbandwerte eingehalten werden.

12. Verfahren nach zumindest einem der vorhergehenden Ansprüche, **dadurch gekennzeichnet, dass** für vorgegebene Werte von Betriebsparametern für ein vorgegebenes Zeitintervall die Bildung des NOX-Sensor-Diagnosesignals und/oder des Temperatur-Diagnosesignals ausgesetzt wird.

13. Verfahren nach zumindest einem der vorhergehenden Ansprüche, **dadurch gekennzeichnet, dass** in Abhängigkeit von den Werten gebildeter Diagnosesignale ein NOX-Sensor-Gesamtdiagnosesignal gebildet wird.

14. Vorrichtung zur Eigendiagnose eines in einer Abgasanlage (20) eines Verbrennungsmotors (10) anordbaren NOX-Sensors (232), welcher zur Messung der NOX-Konzentration eines Abgases eine Regeleinrichtung zur Regelung eines Sauerstoffpartialdrucks in einer ersten Diffusionskammer (232) des NOx-Sensors (23) aufweist und die Regeleinrichtung ein den Lambda-Wert des Abgases charakterisierendes Lambdasignal (U02, UVP) zur Verfügung stellt, und eine NOX-Messeinrichtung aufweist, die ein die NOX-Konzentration im Abgas charakterisierendes NOX-Signal zur Verfügung stellt, wobei eine Diagnoseeinrichtung (40) vorgesehen ist mit Vergleichsmitteln zum Vergleich von ermittelten Werten des Lambdasignals mit bei vorgegebenen Betriebsparametern plausiblen Lambda-Werten des Abgases und zur Bildung eines ersten Vergleichsergebnisses sowie mit Auswertungsmitteln zur Bildung eines ersten NOX-Sensor-Diagnosesignals in Abhängigkeit des ersten Vergleichsergebnisses , **dadurch gekennzeichnet, dass** die Diagnostein nichtung weiterhin vergleichsmittel zum Vergleich von Werten des NOX-Signals mit bei vorgegebenen Betriebsparametern plausiblen Werten des NOX-Signals und zur Bildung eines zweiten Vergleichsergebnisses sowie Auswertungsmittel zur Bildung eines zweiten NOX-Sensor-Diagnosesignals in Abhängigkeit des zweiten Vergleichsergebnisses aufweist

## Claims

1. Method for self-diagnosis of a NOX sensor (23) arranged in an exhaust system (20) of an internal combustion engine (10), which NOX sensor, for measuring the NOX concentration of an exhaust gas, has a regulating device for regulating an oxygen partial pressure in a first diffusion chamber (232) of the NOX sensor (23), and the regulating device outputs a lambda signal (U02, UVP) which characterizes the lambda value of the exhaust gas, and which NOX sensor has a NOX measurement device which outputs a NOX signal which characterizes the NOX concentration in the exhaust gas, wherein determined values of the lambda signal (U02, UVP) are compared with lambda values of the exhaust gas which are plausible for predefined operating parameters, and a first NOX sensor diagnostic signal is formed as a function of the comparison result, **characterized in that**
the values of the NOX signal are compared with values of the NOX concentration which are plausible for predefined operating parameters, and a second NOX sensor diagnostic signal is formed as a function of the comparison result.

2. Method according to Claim 1, **characterized in that** the lambda signal is a broadband lambda signal (U02) or a lambda step signal (UVP).

3. Method according to at least one of the preceding claims, **characterized in that** the regulating device for regulating the oxygen partial pressure is in the form of an oxygen measurement pump cell device (231) which operates on the Nernst principle.

4. Method according to at least one of the preceding claims, **characterized in that** the NOX measurement device is in the form of a NOX measurement pump cell device (237) which operates on the Nernst principle.

5. Method according to at least one of Claims 3 and 4, **characterized in that** the NOX sensor (23) has the first diffusion chamber (232) and a second diffusion chamber (236), to which is assigned in each case one of the measurement pump cell devices (231; 237).

6. Method according to at least one of the preceding claims, **characterized in that** determined values of a regulating signal, which is regulated to a constant value, of the first diffusion chamber (232) are compared with values of the regulating signal which are plausible for predefined operating parameters, and a further NOX sensor diagnostic signal is formed as a function of the comparison result.

7. Method according to at least one of the preceding claims, **characterized in that** the NOX sensor (23) has a heating device (238) with an associated temperature measurement device in order to operate the NOX sensor (23) at a predefined temperature, wherein the temperature measurement device outputs a temperature signal from which the NOX sensor temperature can be determined, and wherein the values of the temperature signal are compared with values of the NOX sensor temperature which are plausible for predefined operating parameters, and a temperature diagnostic signal is formed as a function of the comparison result.

8. Method according to Claim 7, **characterized in that** the heating device (238) has a temperature regulating means and **in that** if, after a predefined regulating time, the value of the temperature signal corresponds to a temperature below a predefined operating temperature of the NOX sensor (23), a temperature diagnostic signal which characterizes a fault is formed.

9. Method according to Claim 7 or 8, **characterized in that** the temperature measurement device operates on the basis of the determination of an internal resistance of the NOX sensor (23).

10. Method according to Claims 1, 6 and 7, **characterized in that** the values of the lambda signal, of the NOX signal, of the regulating signal or of the temperature signal are determined when the NOX sensor is being heated and when the NOX sensor is not-being heated.

11. Method according to Claims 1, 6 and 7, **characterized in that**, to detect line faults, during the comparison of the values of the lambda signal, of the NOX signal, of the regulating signal or of the temperature signal with the respective, plausible values, a check is performed with regard to whether predefined maximum, minimum or tolerance band values are adhered to.

12. Method according to at least one of the preceding claims, **characterized in that**, for predefined values of operating parameters, the formation of the NOX sensor diagnostic signal and/or of the temperature diagnostic signal is suspended for a predefined time interval.

13. Method according to at least one of the preceding claims, **characterized in that** a NOX sensor overall diagnostic signal is formed as a function of the values of formed diagnostic signals.

14. Device for self-diagnosis of a NOX sensor (23) which can be arranged in an exhaust system (20) of an internal combustion engine (10), which NOX sensor, for measuring the NOX concentration of an exhaust gas, has a regulating device for regulating an oxygen partial pressure in a first diffusion chamber (232) of the NOX sensor (23), and the regulating device outputs a lambda signal (U02, UVP) which characterizes the lambda value of the exhaust gas, and which NOX sensor has a NOX measurement device which outputs a NOX signal which characterizes the NOX concentration in the exhaust gas, wherein a diagnostic device (40) is provided which has comparison means for comparing determined values of the lambda signal with lambda values of the exhaust gas which are plausible for predefined operating parameters and for forming a first comparison result, and evaluation means for forming a first NOX sensor diagnostic signal as a function of the first comparison result, **characterized in that** the diagnostic device furthermore has comparison means for comparing values of the NOX signal with values of the NOX signal which are plausible for predefined operating parameters and for forming a second comparison result, and evaluation means for forming a second NOX sensor diagnostic signal as a function of the second comparison result.

## Revendications

1. Procédé d'autodiagnostic d'un capteur de NOx (23) disposé dans un système d'échappement (20) d'un moteur à combustion (10), lequel, pour mesurer la concentration de NOx de gaz d'échappement, présente un dispositif de régulation pour réguler une pression partielle d'oxygène dans une première chambre de diffusion (232) du capteur de NOx (23) et le dispositif de régulation met à disposition un signal Lambda (UO2, UVP, qui caractérise la valeur Lambda des gaz d'échappement, et lequel présente un dispositif de mesure de NOx qui met à disposition un signal de NOx qui caractérise la concentration de NOx dans les gaz d'échappement, les valeurs déterminées du signal Lambda (U02, UVP) étant comparées avec des valeurs Lambda plausibles des gaz d'échappement à des paramètres de fonctionnement prédéfinis et un premier signal de diagnostic du capteur de NOx étant formé en fonction du résultat de la comparaison, **caractérisé en ce que** les valeurs du signal de NOx sont comparées avec les valeurs de la concentration de NOx plausibles aux paramètres de fonctionnement prédéfinis et un deuxième signal de diagnostic du capteur de NOx est formé en fonction du résultat de la comparaison.

2. Procédé selon la revendication 1, **caractérisé en ce que** le signal Lambda est un signal Lambda à large bande (U02) ou un signal carré Lambda (UVP).

3. Procédé selon au moins l'une des revendications précédentes, **caractérisé.en ce que** le dispositif de régulation destiné à réguler la pression partielle d'oxygène est réalisé sous la forme d'un dispositif à cellules de pompe de mesure d'oxygène (231) fonctionnant selon le principe de Nernst.

4. Procédé selon au moins l'une des revendications précédentes, **caractérisé en ce que** le dispositif de mesure de NOx est réalisé sous la forme d'un dispositif à cellules de pompe de mesure de NOx (237) fonctionnant selon le principe de Nernst.

5. Procédé selon au moins l'une des revendications 3 ou 4, **caractérisé en ce que** le capteur de NOx (23) présente la première chambre de diffusion (232) et une deuxième chambre de diffusion (236) qui sont respectivement associées à l'un des dispositifs à cellules de pompe de mesure (231 ; 237).

6. Procédé selon au moins l'une des revendications précédentes, **caractérisé en ce que** les valeurs déterminées d'un signal de régulation régulé à une valeur constante de la première chambre de diffusion (232) sont comparées avec des valeurs plausibles du signal de régulation aux paramètres de fonctionnement prédéfinis et un signal de diagnostic supplémentaire du capteur de NOx est formé en fonction du résultat de la comparaison.

7. Procédé selon au moins l'une des revendications précédentes, **caractérisé en ce que** le capteur de NOx (23) présente un dispositif de chauffage (238) muni d'un dispositif de mesure de la température associé pour faire fonctionner le capteur de NOx (23) à une température prédéfinie, le dispositif de mesure de la température délivrant un signal de température à partir duquel peut être déterminée la température du capteur de NOx et les valeurs du signal de température étant comparées avec des valeurs plausibles de la température du capteur de NOx aux paramètres de fonctionnement prédéfinis et un signal de diagnostic de température étant formé en fonction du résultat de la comparaison.

8. Procédé selon la revendication 7, **caractérisé en ce que** le dispositif de chauffage (238) présente un régulateur de température et **en ce que**, après un temps de régulation prédéfini, si la valeur du signal de température correspond à une température inférieure à une température de fonctionnement prédéfinie du capteur de NOx (23), un signal de diagnostic de température caractérisant un défaut est formé.

9. Procédé selon la revendication 7 ou 8, **caractérisé en ce que** le dispositif de mesure de la température fonctionne selon le principe de la détermination d'une résistance interne du capteur de NOx (23) .

10. Procédé selon les revendications 1, 6 et 7, **caractérisé en ce que** les valeurs du signal Lambda, du signal de NOx, du signal de régulation ou du signal de température sont déterminées avec le capteur de NOx chauffé et non chauffé.

11. Procédé selon les revendications 1, 6 et 7, **caractérisé en ce que** pour détecter des défauts de ligne lors de la comparaison des valeurs du signal Lambda, du signal de NOx, du signal de régulation ou du signal de température avec les valeurs plausibles respectives, un contrôle est effectué pour vérifier si les valeurs prédéfinies du maximum, du minimum ou de la bande de tolérance sont respectées.

12. Procédé selon au moins l'une des revendications précédentes, **caractérisé en ce que** pour des valeurs prédéfinies de paramètres de fonctionnement, la formation du signal de diagnostic du capteur de NOx et/ou du signal de diagnostic de température est suspendue pendant un intervalle de temps prédéfini.

13. Procédé selon au moins l'une des revendications précédentes, **caractérisé en ce qu'**un signal de diagnostic global du capteur de NOx est formé en fonction des valeurs des signaux de diagnostic formés.

14. Dispositif d'autodiagnostic d'un capteur de NOx (23) pouvant être disposé dans un système d'échappement (20) d'un moteur à combustion (10), lequel, pour mesurer la concentration de NOx de gaz d'échappement, présente un dispositif de régulation pour réguler une pression partielle d'oxygène dans une première chambre de diffusion (232) du capteur de NOx (23) et le dispositif de régulation met à disposition un signal Lambda (U02, UVP) qui caractérise la valeur Lambda des gaz d'échappement, et lequel présente un dispositif de mesure de NOx qui met à disposition un signal de NOx qui caractérise la concentration de NOx dans les gaz d'échappement, un dispositif de diagnostic (40) étant prévu, comprenant des moyens de comparaison pour comparer les valeurs déterminées du signal Lambda avec des valeurs Lambda plausibles des gaz d'échappement à des paramètres de fonctionnement prédéfinis et pour former un premier résultat de comparaison, et comprenant aussi des moyens d'interprétation pour former un premier signal de diagnostic du capteur de NOx en fonction du premier résultat de comparaison, **caractérisé en ce que** le dispositif de diagnostic présente en outre des moyens de comparaison pour comparer les valeurs du signal de NOx avec des valeurs du signal de NOx plausibles aux paramètres de fonctionnement prédéfinis et pour former un deuxième résultat de comparaison, ainsi que des moyens d'interprétation pour former un deuxième signal de diagnostic du capteur de NOx en fonction du deuxième résultat de comparaison.
